(19) Europäisches Patentamt
European Patent Office
Office européen des brevets

(11) **EP 3 932 310 B1**

(12) **EUROPEAN PATENT SPECIFICATION**

(45) Date of publication and mention
of the grant of the patent:
**15.10.2025  Bulletin 2025/42**

(21) Application number: **20892276.5**

(22) Date of filing: **19.08.2020**

(51) International Patent Classification (IPC):
**A61B 5/1495** (2006.01)     **A61B 5/145** (2006.01)
**A61B 5/155** (2006.01)     **A61B 5/00** (2006.01)

(52) Cooperative Patent Classification (CPC):
**A61B 5/14532; A61B 5/1495; A61B 5/746;**
A61B 2560/0223; A61B 2560/0238

(86) International application number:
**PCT/KR2020/011006**

(87) International publication number:
**WO 2021/107335 (03.06.2021 Gazette 2021/22)**

(54) **METHOD FOR CALIBRATING BLOOD GLUCOSE VALUE IN CONTINUOUS BLOOD GLUCOSE MEASUREMENT SYSTEM**

VERFAHREN ZUR KALIBRIERUNG DES BLUTZUCKERWERTES IN EINEM KONTINUIERLICHEN BLUTZUCKERMESSSYSTEM

PROCÉDÉ D'ÉTALONNAGE DE VALEUR DE GLYCÉMIE DANS UN SYSTÈME DE MESURE CONTINUE DE LA GLYCÉMIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.11.2019  KR 20190153096**

(43) Date of publication of application:
**05.01.2022  Bulletin 2022/01**

(73) Proprietor: **i-Sens, Inc.**
**Seoul 06646 (KR)**

(72) Inventors:
• **SEO, Jung Hee**
**Seoul 06646 (KR)**
• **NAH, Ji Seon**
**Seoul 06646 (KR)**
• **LEE, David**
**Seoul 06646 (KR)**
• **KANG, Young Jea**
**Seoul 06646 (KR)**

(74) Representative: **BCKIP Part mbB**
**MK1**
**Landsbergerstraße 98, 3.Stock**
**80339 München (DE)**

(56) References cited:
JP-A- 2011 062 335      KR-A- 20180 106 783
KR-A- 20190 010 931     KR-A- 20190 057 759
US-A1- 2011 054 809     US-A1- 2011 184 268
US-A1- 2016 081 597     US-B1- 9 492 118

## Description

### TECHNICAL FIELD

Some exemplary embodiments of the present disclosure relate to a method of calibrating a blood glucose value in a continuous blood glucose measurement system, more specifically, a method in which a blood glucose value can be calibrated precisely by selecting a different calibration mode calibrating a blood glucose value based on whether a difference between a blood glucose value measured by a continuous blood glucose measurement system and a reference blood glucose value measured by a separate blood glucose measurement device is out of a predetermined range, and the blood glucose value measured by a continuous blood glucose measurement system can be calibrated precisely by activating a scroll such that a reference blood glucose value can be inputted to an input window only within a reference blood glucose value range calculated from the blood glucose value measured by the continuous blood glucose measurement system and a predetermined threshold range or, when a reference blood glucose value which is out of the reference blood glucose range is inputted, calibrating a blood glucose value with a second calibration mode forceably or automatically using a plurality of reference blood glucose values.

### BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

Because blood glucose needs to be measured steadily in order to manage diabetes, the needs of devices related to blood glucose measurement are constantly increasing. When diabetes patients strictly manage blood glucose control, various researches confirmed that occurrences of complications of diabetes are significantly reduced. Accordingly, it is important for diabetes patients to regularly measure blood glucose for blood glucose management.

Generally, a blood-collecting type blood glucose measurement device (finger prick method) is mainly used for blood glucose management of diabetes patients, and the blood-collecting type blood glucose measurement device can help the blood glucose management of the diabetes patients, but there is a problem that it is difficult of figuring out a precise blood glucose level because the result shows a result at the time of measurement only. Additionally, as the blood-collecting type blood glucose measurement device requires to collect blood several times in a day in order to measure blood glucose, the burden of finger prick for collecting blood to diabetes patients is a problem.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous glucose monitoring system includes a sensor transmitter attached to a body part of a user and measuring a blood glucose level by extracting body fluid, a communication terminal outputting the received blood glucose level, and so on. The sensor transmitter continuously measures the blood glucose of a user in a status that a sensor is inserted to a human body for a certain period, for example, 15 days, and generates blood glucose information periodically.

An application for blood glucose management is installed at the communication terminal, and the communication terminal periodically receives and outputs the blood glucose information from the sensor transmitter so that the user can check the received blood glucose information.

In the continuous blood glucose measurement system, one to three hours of a stabilization step may be required when the sensor transmitter is inserted into a body part of a user, and when the stabilization is completed, the communication terminal displays the blood glucose information received from the sensor transmitter on a display unit and provides it to the user.

In order to provide precise blood glucose information to a user, the blood glucose information received from the sensor transmitter needs to be calibrated at an early stage, and after that it needs to be continuously calibrated at a certain cycle for a use time period of the sensor transmitter. During the calibration at the early stage, a reference blood glucose value measured by a separate blood glucose measurement device is inputted to the communication terminal, the blood glucose information received from the sensor transmitter is calibrated with the reference blood glucose value, and after that blood glucose information of the sensor transmitter needs to be calibrated continuously with the reference blood glucose value measured through the blood glucose measurement device at a certain cycle for a use time period of the sensor transmitter.

However, when a difference between a blood glucose value measured through a continuous blood glucose measurement system and a reference blood glucose value measured through a separate blood glucose measurement device exceeds a predetermined range, a single reference blood glucose value is not enough to calibrate a blood glucose value because the credibility of the reference blood glucose value or the blood glucose value is low and it is not safe to calibrate the blood glucose value based on a single reference blood glucose value. Accordingly, when a difference between a blood glucose value measured through a continuous blood glucose measurement system and a reference blood glucose value measured through a separate blood glucose measurement device is out of a predetermined range, a technique for calibrating a blood glucose value measured by a continuous blood glucose measurement system is needed. US 2016/0081597 Al discloses systems and methods for providing dynamic and intelligent ways to change the required level of user interaction during use of an analyte monitoring device. The analyte monitoring device may transition or switch types of calibration modes from a user-dependent calibration mode to a device self-calibration mode. The user-dependent calibration mode is a calibration routine using blood glucose concentration values from an external meter. The device self-calibration mode is a calibration routine performed by the device without requiring a user to provide a glucose value from an external meter. Device self-calibration generally requires one or more of stringent manufacturing controls, internal measurements, entry of calibration codes from the manufacturer (which themselves constitute a priori information) and/or associated algorithms that enable calibration of the device without external measurement obtained during sensor wear.

## SUMMARY

### Technical Problem

Certain embodiments of the present disclosure solve the above-mentioned problems of a calibration method of a blood glucose value in the conventional continuous blood glucose measurement system, and the purposes of some embodiments of the present disclosure are for providing a method for precisely calibrating a blood glucose value by selecting a different calibration mode for calibrating a blood glucose value based on whether a difference between a blood glucose value measured by a continuous blood glucose measurement system and a reference blood glucose value measured by a separate blood glucose measurement device is out of a predetermined range.

Another purpose of certain embodiments of the present disclosure is for providing a blood glucose value calibration method for activating a scroll such that a reference blood glucose value can be inputted to an input window only within a reference blood glucose value range calculated from the blood glucose value measured by a blood glucose measurement device and a predetermined threshold range and, when a reference blood glucose value which is out of the reference blood glucose range is inputted, calibrating a blood glucose value with a second calibration mode forceably or automatically using a plurality of reference blood glucose values.

Still another purpose of certain embodiments of the present disclosure is for providing a blood glucose value calibration method for alarming to a user whether a sensor transmitter is properly attached to a human body based on a difference between a blood glucose value measured at a calibration period and a refence blood glucose value or, when a time difference between times inputting a plurality of reference blood glucose values is significantly long, alarming to input a reference blood glucose value consecutively measured.

### Solution to Problem

For accomplish the purpose of the present disclosure, according to an embodiment of the present disclosure, a method of calibrating a blood glucose value measured by a continuous blood glucose measurement device comprises: retrieving information of a blood glucose value measured by a continuous blood glucose measurement device at a calibration period; measuring a reference blood glucose value using a separate blood glucose measurement device which is a separate device from the continuous blood glucose measurement device at the calibration period; calculating a difference between the reference blood glucose value and the blood glucose value of the continuous blood glucose measurement device; selecting one of a first calibration mode and a second calibration mode as a calibration mode for calibrating the blood glucose value of the continuous blood glucose measurement device based on whether or not the difference between the reference blood glucose value and the blood glucose value of the continuous blood glucose measurement device is out of a threshold range; and calibrating the blood glucose value of the continuous blood glucose measurement device using the reference blood glucose value according to the selected calibration mode. When the difference between the blood glucose value of the continuous blood glucose measurement device and the reference blood glucose value measured by the separate blood glucose measurement device is within a first threshold range, the first calibration mode is selected and the blood glucose value of the continuous blood glucose measurement device is calibrated using the reference blood glucose value at the first calibration mode. When the difference between the blood glucose value of the continuous blood glucose measurement device and the reference blood glucose value measured by the separate blood glucose measurement device is out of the first threshold range, the second calibration mode is selected and the blood glucose value of the continuous blood glucose measurement device is calibrated using a calibration value calculated from a plurality of reference blood glucose values measured by the separate blood glucose measurement device at the second calibration mode.

Here, the information of the blood glucose value may be a blood glucose value or a blood glucose change value.

Preferably, the method of calibrating the blood glucose value according to an embodiment of the present disclosure may further comprise: calculating a range of an inputtable reference blood glucose value, which is inputtable at the first calibration mode, based on the blood glucose value measured by the continuous blood glucose measurement device at the calibration period and the first threshold range; and control to activate a scroll for inputting the reference blood glucose value within the calculated range of the inputtable reference blood glucose value on a user interface screen, wherein the reference blood glucose value is inputted through the activated scroll.

Here, a user command for selecting the second calibration mode may be inputted through the user interface screen if the reference blood glucose value is out of the range of the inputtable reference blood glucose value of the activated scroll.

Here, the plurality of the refence blood glucose values measured at different times are inputted through the user interface screen at the second calibration mode.

Preferably, the selecting of one of the first calibration mode and the second calibration mode comprises: activating an input window for inputting the reference blood glucose measured at the calibration period; determining whether the difference between the blood glucose value of the continuous blood glucose measurement device and the reference blood glucose value measured by the separate blood glucose measurement device is out of the first threshold range; and if the difference between the blood glucose value of the continuous blood glucose measurement device and the reference blood glucose value measured by the separate blood glucose measurement device is within the first threshold range, selecting the first calibration mode as the calibration mode, and if the difference between the blood glucose value of the continuous blood glucose measurement device and the reference blood glucose value measured by the separate blood glucose measurement device is out of the first threshold range, selecting the second calibration mode as the calibration mode.

Here, when the difference between the blood glucose value of the continuous blood glucose measurement device and the reference blood glucose value measured by the separate blood glucose measurement device is out of a second threshold range, outputting an alarm to a user is provided.

Here, monitoring times in which the plurality of reference blood glucose values are inputted at the second calibration mode, and when a difference between the times in which the plurality of reference blood glucose values are inputted exceeds a predetermined threshold time difference, outputting an alarm to a user may be provided.

**Advantageous Effects of Invention**

A method of calibrating a blood glucose value in a continuous blood glucose measurement system according to various embodiments of the present disclosure may have following technical effects.

Firstly, a blood glucose value calibration method according to an embodiment of the present disclosure can precisely calibrate a blood glucose value according to a calibration mode by selecting a different calibration mode for calibrating a blood glucose value based on whether a difference between a blood glucose value measured by a continuous blood glucose measurement system and a reference blood glucose value measured by a separate blood glucose measurement device is out of a predetermined range.

Secondly, a blood glucose value calibration method according to an embodiment of the present disclosure can accurately calibrate a blood glucose value of a user by activating a scroll such that a reference blood glucose value can be inputted to an input window only within a reference blood glucose value range calculated from the blood glucose value measured by a blood glucose measurement device and a predetermined threshold range, or, when a reference blood glucose value which is out of the reference blood glucose range is inputted, calibrating a blood glucose value with a second calibration mode forceably or automatically using a plurality of reference blood glucose values.

Thirdly, a blood glucose value calibration method according to an embodiment of the present disclosure can accurately calibrate a blood glucose value of a user by alarming to a user whether a sensor transmitter is properly attached to a human body based on a difference between a blood glucose value measured at a calibration period and a refence blood glucose value or, when a time difference between times inputting a plurality of reference blood glucose values is significantly long, alarming to input a reference blood glucose value consecutively measured.

## BRIEF DESCRIPTION OF THE DRAWINGS

FIG. 1 is a schematic diagram for illustrating a continuous blood glucose measurement system according to an embodiment of the present disclosure.

FIG. 2 is a drawing for illustrating an example of inputting initial calibration information and periodic calibration information.

FIG. 3 is a drawing for illustrating an apparatus of calibrating a value of blood glucose according to an embodiment of the present disclosure.

FIG. 4 is a flow chart for illustrating a method of calibrating a blood glucose value of a continuous blood glucose measurement device according to an embodiment of the present disclosure.

FIG. 5 is a flow chart for illustrating a method of selecting a calibration mode according to an embodiment of the present disclosure.

FIG. 6 illustrates an example of a user interface screen which is displayed on a user terminal to select a calibration mode according to an embodiment of the present disclosure.

FIG. 7 is a flow chart for illustrating a method of selecting a calibration mode according to another embodiment of the present disclosure.

FIG. 8 illustrates an example of a user interface screen displayed on a user terminal for selecting a calibration mode according to another embodiment of the present disclosure.

FIG. 9 is a flow chart for illustrating a method for providing an alarm to a user based on a reference blood glucose value inputted through a user interface screen according to an embodiment of the present disclosure.

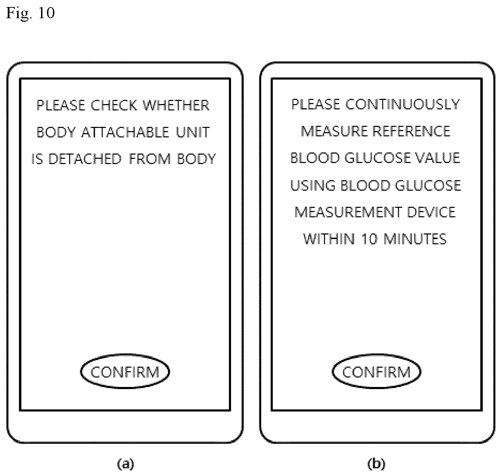

FIG. 10 illustrates an example of a user interface screen providing an alarm according to a rotation inquiry command.

## DETAILED DESCRIPTION OF EMBODIMENTS

The technical terms used in the present disclosure are only for the purpose of describing exemplary embodiments, and they are not intended to limit the present invention. Also, unless otherwise defined, all technical terms used herein should be construed as having the same meaning as commonly understood by those skilled in the art, and should not be interpreted as being excessively inclusive or excessively restrictive.

Additionally, singular expressions used in the present disclosure include plural meaning unless the context clearly dictates otherwise. In the present disclosure, the terms "comprising", "having", "including" and the like should not be construed to necessarily include all component elements or steps described in the present disclosure, and it should be interpreted that some component elements or some steps among them may not be included, or other components or steps may be added thereto.

Also, it should be noted that the accompanying drawings are merely illustrated to easily explain the invention, and therefore, they should not be construed as limiting.

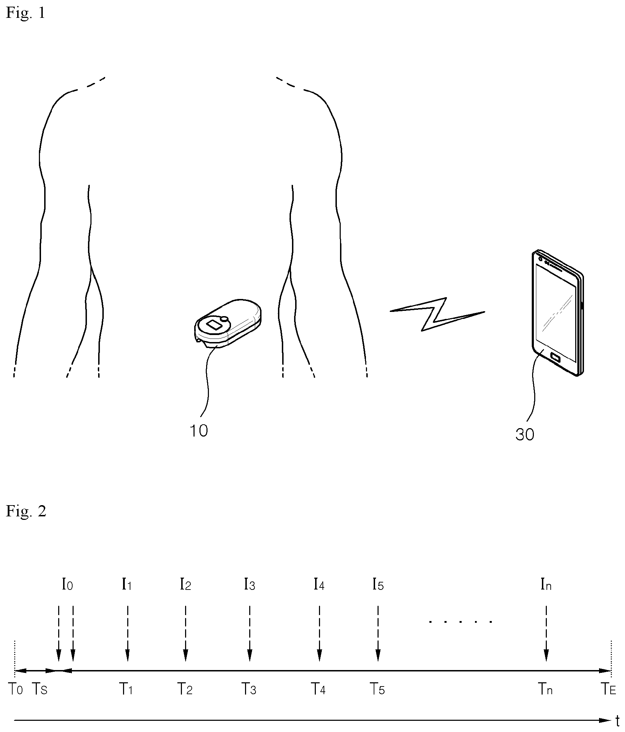

FIG. 1 is a schematic diagram for illustrating a continuous blood glucose measurement system according to an embodiment of the present disclosure.

Referring to FIG. 1, the continuous blood glucose measurement system according to an embodiment of the present disclosure comprises a sensor transmitter (10) and a communication terminal (30).

The sensor transmitter (10) is attachable to a human body, and when the sensor transmitter (10) is attached to the human body, one end portion of the sensor transmitter (10) is inserted into a skin, and periodically extracts the body fluid of the human body and continuously measures blood glucose.

The communication terminal (30) is a terminal configured to receive information on blood glucose from the sensor transmitter (10), and for example, a portable terminal which is capable of communicating with the sensor transmitter (10) such as a smartphone, tablet PC, or notebook can be used. Of course, the communication terminal (30) is not limited thereto, and the communication terminal (30) can be any type of a terminal which can have a communication function and a program or application can be installed in.

The sensor transmitter (10) transmits to the communication terminal (30) information related to blood glucose measured pursuant to a request of the communication terminal (30) or periodically at every predetermined time period, and for data communication between the sensor transmitter (10) and the communication terminal (30), the sensor transmitter (10) and the communication terminal (30) are communicationally connected to each other through wired communication such as a USB cable and so on or through wireless communication such as infrared light communication, NFC communication, Bluetooth communication, and the like.

More specifically, when the communication between the sensor transmitter and the communication terminal is connected, after the transmitter is stabilized, a blood glucose value measured by the sensor transmitter is initially calibrated using a calibration information which is a reference value of blood glucose measured by a separate blood glucose measurement device. After that, the blood glucose level received from the sensor transmitter is calibrated using the calibration information and the calibrated blood glucose value is provided to be outputted to the user.

In order to precisely calibrate the blood glucose information measured by the sensor transmitter, the calibration is performed using new calibration information to which a reference value of blood glucose measured by a separate blood glucose measurement device periodically for a period of using the sensor transmitter is set, the blood glucose value received from the sensor transmitter is calibrated using the new calibration information and the calibrated blood glucose level is provided to be outputted to the user.

FIG. 2 is a drawing for illustrating an example of inputting initial calibration information and periodic calibration information, and referring to FIG. 2, the sensor transmitter is stabilized until set stabilization time (Ts) is elapsed from time $(T_0)$ when the sensor transmitter and the communication terminal are communicationally connected.

When the stabilization of the transmitter is completed, an initial calibration information $(I_0)$ is inputted. Here, the initial calibration information $(I_0)$ can be inputted multiple times to precisely calibrate the biometrics information of the sensor transmitter.

New calibration information $(I_1, I_2, I_3, I_4, ...)$ is inputted after the stabilization of the sensor transmitter is completed until expiration time $(T_E)$ of period of use, preferably every 12 hour or every day, and the blood glucose information received from the sensor transmitter is calibrated using the calibration information and the calibrated blood glucose value is provided to the user.

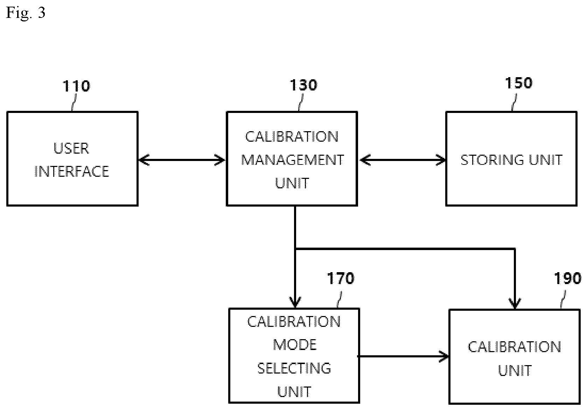

FIG. 3 is a drawing for illustrating an apparatus of calibrating a value of blood glucose according to an embodiment of the present disclosure.

The apparatus of calibrating the value of blood glucose according to an embodiment of the present disclosure can be implemented as a user terminal such as a smartphone.

Referring to FIG. 3, a calibration management unit (130) counts periodic calibration time, after previous calibration time, determines whether next period of the calibration time is reached, and when the next period of the calibration time is reached, control the output of a user interface display for inputting calibration information through a user interface unit (110).

According to an embodiment of the present disclosure, the calibration management unit (130) may be configured to, when the next period of the calibration time is reached, extract from a storing unit or storage (150) a blood glucose value received from the sensor transmitter at the next period of the calibration time, calculate a reference range for blood glucose by considering the extracted blood glucose value and a threshold range, and generate an user interface display such that a reference blood glucose value can be scrolled within the reference range for blood glucose calculated by a input window.

A calibration mode selecting unit (170) selects one of first and second calibration modes as a calibration mode for calibrating a blood glucose value based on whether a difference between a reference blood glucose value inputted through a user interface screen and a blood glucose value is out of a threshold range. For example, the first calibration mode may be a calibration mode calibrating a blood glucose value using a reference blood glucose level based on one inputted reference blood glucose value as calibration information when the difference between the reference blood glucose value and the blood glucose value is within a first threshold range, and the second calibration mode may be a calibration mode calibrating a blood glucose value using calibration information generated based on a plurality of inputted reference blood glucose levels when the difference between the reference blood glucose value and the blood glucose value is out of the first threshold range.

According to the selected calibration mode, a calibration unit (190) calibrats a blood glucose value until a next calibration period by using one reference blood glucose value as calibration information and output it to the user, or a blood glucose value until a next calibration period by using a plurality of reference blood glucose values as calibration information and output it to the user.

Preferably, a calibration management unit (130) is configured to, when a difference between a reference blood glucose value and a blood glucose value exceeds a second threshold range, generate an alarm requesting the user to check an operation status or an arrangement status of a sensor transmitter to the user and output the alarm through the user interface unit (110), or, if an input time difference of reference blood glucose values exceeds a predetermined threshold time difference when generating calibration information by using a plurality of reference blood glucose values, generate an alarm requesting the user to continuously input reference blood glucose values and output the alarm through the user interface (110).

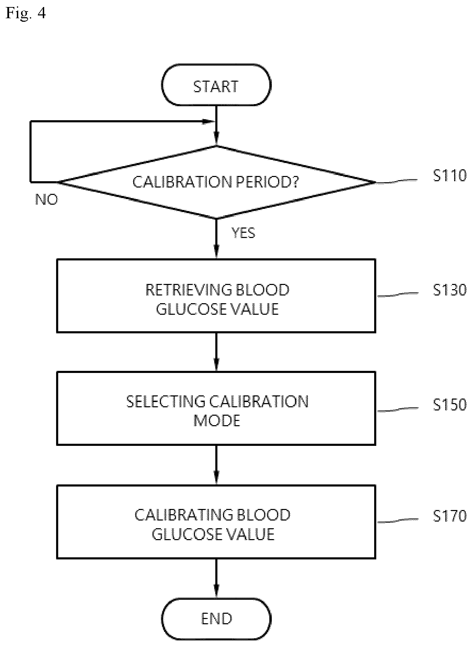

FIG. 4 is a flow chart for illustrating a method of calibrating a blood glucose value of a continuous blood glucose measurement device according to an embodiment of the present disclosure.

Referring to FIG. 4, whether a set calibration period is reached is determined (S110). Here, the calibration period can be set to 12 hours, 1 day or 1 week, and so on, but the calibration period can be fixedly set or be changeably settable by the user.

When the set calibration period is reached, a blood glucose value of the user measured by the sensor transmitter during the corresponding calibration period is retrieved from a storing unit (S130). The sensor transmitter extracts body fluid of the user in real time and periodically transmits a blood glucose value of the user measured based on the extracted body fluid, and the user terminal stores the blood glucose value received from the sensor transmitter to the storing unit.

Based on a blood glucose value retrieved at the calibration period and a reference blood glucose value measured through a test strip by a separate blood glucose measurement device, when a difference between the blood glucose value and the reference blood glucose value is within a threshold range, a first calibration mode is selected as a calibration mode for calibrating a blood glucose level, and when the difference between the blood glucose value and the reference blood glucose value is out of the threshold range, a second calibration mode is selected as a calibration mode for calibrating a blood glucose level (S150).

Calibration information is generated based the selected calibration mode and the blood glucose value received from the sensor transmitter is calibrated using the generated calibration information (S170). In this operation, when the first calibration mode is selected as a calibration mode, a blood glucose value is calibrated based on a reference blood glucose value by using the measured reference blood glucose value as calibration information, and when the second calibration mode is selected as a calibration mode, calibration information is generated based on a plurality of reference blood glucose values and a blood glucose value by inputting a plurality of reference blood glucose values measured at different time from each other and a blood glucose value is calibrated using the generated calibration information.

An example of generating calibration information in the second calibration mode according to a field to which the present disclosure is applied is a mathematical formula (1) as follows.

[Mathematical Formula 1]

$$V_A = \omega_1 \times V_{St_1} + \omega_2 \times V_{St_2} + \omega_3 \times V_{t_1}$$

$$( \omega_1 + \omega_2 + \omega_3 = 1 )$$

Here, $V_A$ is calibration information used in the second calibration mode, $V_{st1}$, $V_{st2}$ are reference blood glucose values measured by a separate blood glucose measuring device at time t1 and t2, respectively, $V_{t1}$ is a blood glucose value measured at time t1 through a sensor transmitter, and $\omega_1$, $\omega_2$ and $\omega_3$ are weights assigned to $V_{St1}$, $V_{St2}$, $V_{t1}$, respectively. The second calibration mode is generated in the second calibration mode by using two or more reference blood glucose values according to a field to which the present disclosure is applied.

Additionally, the times of measuring the blood glucose value and the reference blood glucose value may be different from each other.

By using calibration information calculated from the blood glucose value and the reference blood glucose value at a calibration period, a blood glucose value received from a sensor transmitter is calibrated and provided to a user until a next calibration period is start.

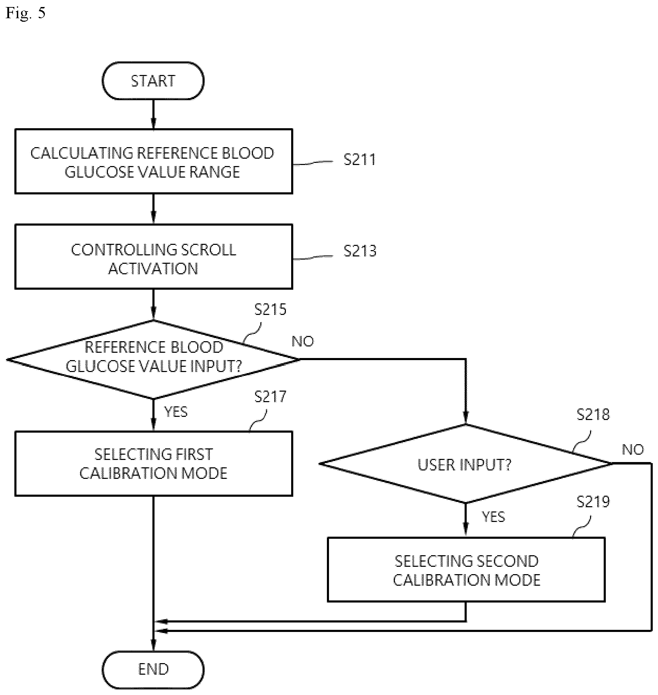

FIG. 5 is a flow chart for illustrating a method of selecting a calibration mode according to an embodiment of the present disclosure.

Referring to FIG. 5, a reference blood glucose value range is calculated based on a blood glucose value measured by a sensor transmitter at a calibration period (S211). For example, the reference blood glucose value range is calculated by adding a threshold range to the blood glucose value to calculate an upper limit value of the reference blood glucose value range and subtracting a threshold range from the blood glucose value to calculate a lower limit value of the reference blood glucose value range.

When a reference blood glucose value is inputted into an input window for inputting a reference blood glucose value of a calibration period, the reference blood glucose value to be inputted can be scrolled only within the calculated reference blood glucose value range, and therefore the activation of the input window can be controlled (S213).

Whether the reference blood glucose value is inputted through the input window is determined (S215), and when the reference blood glucose value is inputted through the input window, for example, when the reference blood glucose value is inputted by scrolling the reference blood glucose range at the input window, the first calibration mode is selected for a calibration mode for calibrating a blood glucose value (S217).

However, when the reference blood glucose value cannot be inputted through the input window, for example, when the reference blood glucose value is out of a scrollable reference blood glucose range of the input window, whether a user command for manually inputting the reference blood glucose value is inputted is determined (S218). When a plurality of reference blood glucose levels are inputted continuously through the user command, the second calibration mode is selected for a calibration mode for calibrating a blood glucose value (S219).

Accordingly, the scrollable range is activation-controlled so that a reference blood glucose value can be inputted only within a reference blood glucose range, and when the reference blood glucose value to be inputted is out of the scrollable reference blood glucose range, the calibration mode can be forceably or manually changed to the second calibration mode so that the blood glucose value can be calibrated precisely by using the plurality of the reference blood glucose values.

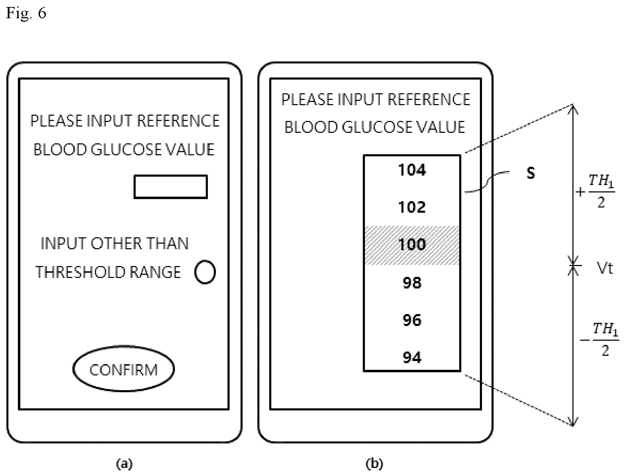

FIG. 6 illustrates an example of a user interface screen which is displayed on a user terminal to select a calibration mode according to an embodiment of the present disclosure.

As illustrated in FIG. 6(a), an input window for inputting a reference blood glucose value measured by a separate blood glucose device at a calibration period is activated.

As shown in FIG. 6(b), an input window provides scrollable numbers only within a reference blood glucose range so that a reference blood glucose value can be inputted, an upper limit value of the reference blood glucose range can be calculated by adding a threshold range ($TH_1/2$) to a blood glucose value (Vt) measured at a calibration period and a lower limit value of the reference blood glucose range can be calculated by subtracting a threshold range ($TH_1/2$) from a blood glucose value (Vt) measured at a calibration period. A user can input a reference blood glucose value by scrolling blood glucose values of the input window when the reference blood glucose value is within a reference blood glucose value range.

According to a field to which the present disclosure is applied, a threshold which is used for calculating an upper limit of a reference blood glucose value range and a threshold which is used for calculating a lower limit of a reference blood glucose value range can be different from each other.

However, when the reference blood glucose value cannot be inputted because the reference blood glucose value is out of a scrollable reference blood glucose value range of the input window, a user command for inputting a reference blood glucose value which is out of a threshold range is inputted. In this case, a separate input window for manually inputting or typing a reference blood glucose value by a user is activated, and the user inputs a plurality of reference blood glucose values measured at different times to an input window which is separately activated.

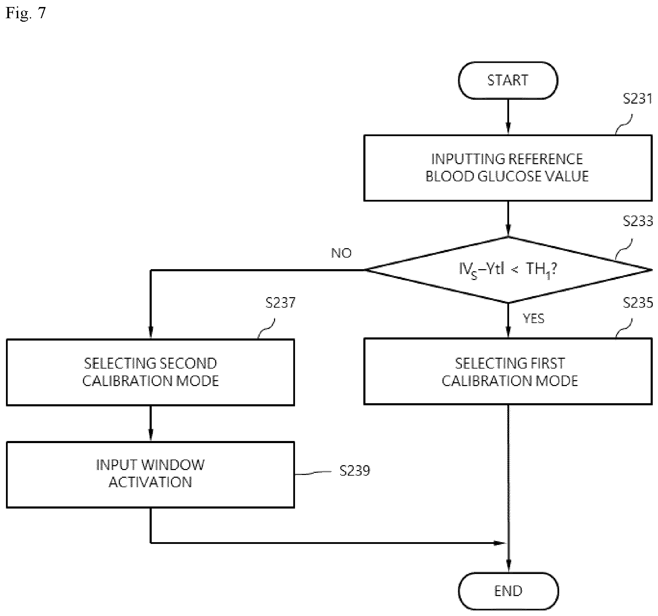

FIG. 7 is a flow chart for illustrating a method of selecting a calibration mode according to another embodiment of the present disclosure.

Referring to FIG. 7, when an input window for inputting a reference blood glucose value measured at a calibration period is activated, the reference blood glucose value measured at the calibration period is inputted through the input window (S231).

By determining whether a difference between a reference blood glucose value (Vs) inputted through the input window and a blood glucose value (Vt) is within a first threshold range ($TH_1$), when the difference between the reference blood glucose value (Vs) and the blood glucose value (Vt) is within the first threshold range ($TH_1$), a first calibration mode is selected for a calibration mode for calibrating a blood glucose value (S235).

However, when the difference between the reference blood glucose value (Vs) and the blood glucose value (Vt) is out of the first threshold range ($TH_1$), a second calibration mode is selected for a calibration mode for calibrating a blood glucose value (S237). When the second calibration mode is selected, a separate input window for inputting a plurality of reference blood glucose values each measured at different times is activated (S239).

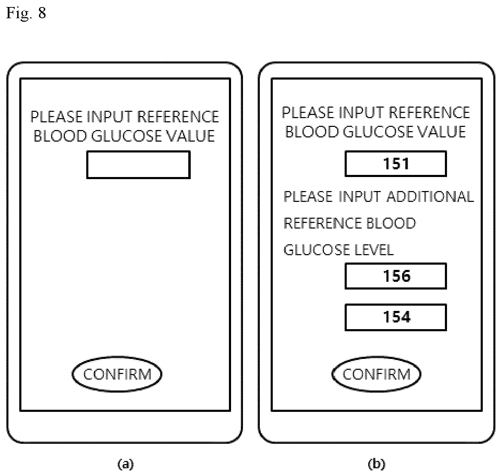

FIG. 8 illustrates an example of a user interface screen displayed on a user terminal for selecting a calibration mode according to another embodiment of the present disclosure.

As illustrated in FIG. 8(a), an input window for inputting a reference blood glucose value measured at a calibration period is activated.

As illustrated in FIG. 8(b), when a difference between a reference blood glucose value inputted to an input window and a blood glucose value is out of a threshold range, a separate input window for inputting a plurality of reference blood glucose values measured at difference times is activated. According to a field to which the present disclosure is applied, at least one or more separate input windows can be activated. When the plurality of reference blood glucose values are inputted, calibration information is generated using the plurality of reference blood glucose values and a blood glucose value is calibrated using the calibration information.

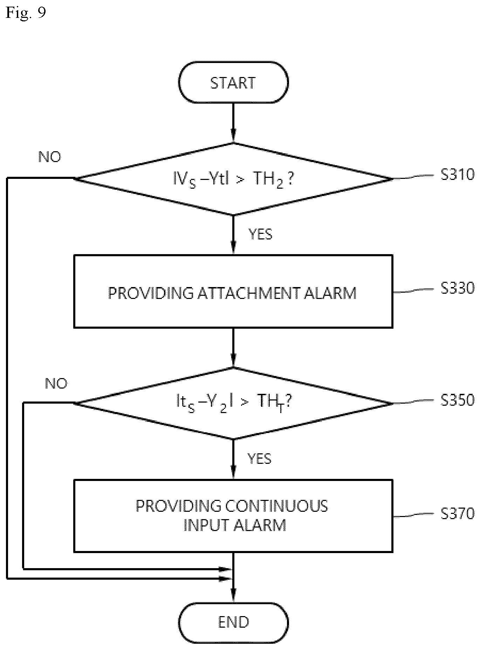

FIG. 9 is a flow chart for illustrating a method for providing an alarm to a user based on a reference blood glucose value inputted through a user interface screen according to an embodiment of the present disclosure.

Referring to FIG. 9, whether or not a difference between a reference blood glucose value inputted through an input window and a blood glucose value is out of a second threshold range is determined (S310). For example, the second threshold range is greater than the first threshold range.

When the difference between the reference blood glucose value inputted through the input window and the blood glucose value is out of the second threshold range, an alarm for checking whether a sensor transmitter is operated normally or separated from a human body is provided to a user (S330). When the difference between the blood glucose value measured from the sensor transmitter and the reference blood glucose value is significantly large, this means that the sensor transmitter is not operated normally, for example, the sensor transmitter malfunctions or is separated from the human body, and in this case, an alarm for checking an wearing or attachment status of the sensor transmitter or an operation status of the sensor transmitter is provided to the user.

Meanwhile, in the second calibration mode, the plurality of reference blood glucose values are inputted, and whether or not a difference between time ($t_1$) in which a first reference blood glucose value is inputted and time ($t_2$) in which a second reference blood glucose value is inputted exceeds a threshold time ($TH_T$) is determined (S350), and when the difference between the time ($t_1$) in which the first reference blood glucose value is inputted and the time ($t_2$) in which the second reference blood glucose value is inputted exceeds the threshold time ($TH_T$), an alarm for continuously inputting reference blood glucose levels measured within a threshold time range is provided to the user (S370). By this alarm, the user can input the reference blood glucose values continuously measured within a certain time range at the second calibration mode so that precise calibration can be performed.

Meanwhile, the exemplary embodiments of the present disclosure described above can be implemented through programs executable at computers, and can be operated in a general-purpose digital computer executing the programs using computer readable medium.

The above-referenced computer readable medium comprises storage medium such as magnetic storage media (e.g., ROM, floppy disks, hard disks, etc.), optical recording media (e.g., CD-ROMs, DVDs, etc.), and carrier waves (e.g., transmission through the Internet).

Although the present disclosure is described with reference to embodiments shown in the drawings in order to explain certain principles of the present disclosure by way of example, a person having ordinary skill in the art which the present disclosure relates could make various modifications and equivalent other embodiments. Accordingly, the protection scope of the present disclosure shall be defined by the claims attached hereto.

**Claims**

1. A method of calibrating a blood glucose value measured by a continuous blood glucose measurement device, the method comprising:

   retrieving (S130) information of a blood glucose value measured by a continuous blood glucose measurement device at a calibration period;
   measuring a reference blood glucose value using a separate blood glucose measurement device which is a separate device from the continuous blood glucose measurement device at the calibration period;
   calculating a difference between the reference blood glucose value and the blood glucose value of the continuous blood glucose measurement device;
   selecting (S150) one of a first calibration mode and a second calibration mode as a calibration mode for calibrating the blood glucose value of the continuous blood glucose measurement device based on whether or not the difference between the reference blood glucose value and the blood glucose value of the continuous blood glucose measurement device is out of a first threshold range; and
   calibrating (S170) the blood glucose value of the continuous blood glucose measurement device using the reference blood glucose value according to the selected calibration mode,
   wherein, when the difference between the blood glucose value of the continuous blood glucose measurement device and the reference blood glucose value measured by the separate blood glucose measurement device is within the first threshold range, the first calibration mode is selected and the blood glucose value of the continuous blood glucose measurement device is calibrated using the reference blood glucose value at the first calibration mode,
   wherein, when the difference between the blood glucose value of the continuous blood glucose measurement device and the reference blood glucose value measured by the separate blood glucose measurement device is out of the first threshold range, the second calibration mode is selected and the blood glucose value of the continuous blood glucose measurement device is calibrated using a calibration value calculated from a plurality of reference blood glucose values measured by the separate blood glucose measurement device at the second calibration mode.

2. The method of calibrating the blood glucose value according to claim 1, further comprising:

   calculating (S211) a range of an inputtable reference blood glucose value, which is inputtable at the first calibration mode, based on the blood glucose value measured by the continuous blood glucose measurement device at the calibration period and the first threshold range; and
   controlling (S213) activation of a scroll for inputting the reference blood glucose value within the calculated range of the inputtable reference blood glucose value on a user interface screen,
   wherein the reference blood glucose value is inputted through the activated scroll.

3. The method of calibrating the blood glucose value according to claim 2, wherein a user command for selecting the second calibration mode is inputted through the user interface screen if the reference blood glucose value is out of the range of the inputtable reference blood glucose value of the activated scroll.

4. The method of calibrating the blood glucose value according to claim 3, wherein the plurality of the reference blood glucose values measured at different times are inputted through the user interface screen at the second calibration mode.

5. The method of calibrating the blood glucose value according to any one of claims 1 to 4, wherein the selecting (S150) of one of the first calibration mode and the second calibration mode comprises:

   activating (S231) an input window for inputting the reference blood glucose measured at the calibration period;
   determining (S233) whether the difference between the blood glucose value of the continuous blood glucose measurement device and the reference blood glucose value measured by the separate blood glucose measurement device is out of the first threshold range; and
   if the difference between the blood glucose value of the continuous blood glucose measurement device and the reference blood glucose value measured by the separate blood glucose measurement device is within the first threshold range, selecting (S235) the first calibration mode as the calibration mode, and if the difference between the blood glucose value of the continuous blood glucose measurement device and the reference blood glucose value measured by the separate blood glucose measurement device is out of the first threshold range, selecting (S237) the second calibration mode as the calibration mode.

6. The method of calibrating the blood glucose value according to any one of claims 2 to 5, further comprising, when the difference between the blood glucose value of the continuous blood glucose measurement device and the reference blood glucose value measured by the separate blood glucose measurement device is out of a second threshold range, outputting (S330) an alarm to a user.

7. The method of calibrating the blood glucose value according to any one of claims 2 to 6, further comprising:

   monitoring times in which the plurality of reference blood glucose values are inputted at the second calibration mode, and
   when a difference between the times in which the plurality of reference blood glucose values are inputted exceeds a predetermined threshold time difference, outputting an alarm to a user.

**Patentansprüche**

1. Verfahren zur Kalibrierung eines Blutzuckerwerts, der von einer Vorrichtung zur kontinuierlichen Blutzuckermessung gemessen wird, wobei das Verfahren umfasst:

   Abrufen (S130) von Informationen über einen Blutzuckerwert, der von einer Vorrichtung zur kontinuierlichen Blutzuckermessung in einem Kalibrierungszeitraum gemessen wird;
   Messen eines Referenzblutzuckerwerts unter Verwendung einer separaten Blutzuckermessvorrichtung, die eine von der Vorrichtung zur kontinuierlichen Blutzuckermessung separate Vorrichtung ist, in dem Kalibrierungszeitraum;
   Berechnen einer Differenz zwischen dem Referenzblutzuckerwert und dem Blutzuckerwert der Vorrichtung zur kontinuierlichen Blutzuckermessung;
   Auswählen (S150) eines ersten Kalibrierungsmodus oder eines zweiten Kalibrierungsmodus als Kalibrierungsmodus zur Kalibrierung des Blutzuckerwerts der Vorrichtung zur kontinuierlichen Blutzuckermessung basierend darauf, ob die Differenz zwischen dem Referenzblutzuckerwert und dem Blutzuckerwert der Vorrichtung zur kontinuierlichen Blutzuckermessung außerhalb eines ersten Schwellenwertbereichs liegt oder nicht; und Kalibrieren (S170) des Blutzuckerwerts der Vorrichtung zur kontinuierlichen Blutzuckermessung unter Verwendung des Referenzblutzuckerwerts gemäß dem ausgewählten Kalibrierungsmodus,

wobei, wenn die Differenz zwischen dem Blutzuckerwert der Vorrichtung zur kontinuierlichen Blutzuckermessung und dem Referenzblutzuckerwert, der von der separaten Blutzuckermessvorrichtung gemessen wird, innerhalb des ersten Schwellenwertbereichs liegt, der erste Kalibrierungsmodus ausgewählt wird und der Blutzuckerwert der Vorrichtung zur kontinuierlichen Blutzuckermessung im ersten Kalibrierungsmodus unter Verwendung des Referenzblutzuckerwerts kalibriert wird,

wobei, wenn die Differenz zwischen dem Blutzuckerwert der Vorrichtung zur kontinuierlichen Blutzuckermessung und dem Referenzblutzuckerwert, der von der separaten Blutzuckermessvorrichtung gemessen wird, außerhalb des ersten Schwellenwertbereichs liegt, der zweite Kalibrierungsmodus ausgewählt wird und der Blutzuckerwert der Vorrichtung zur kontinuierlichen Blutzuckermessung im zweiten Kalibrierungsmodus unter Verwendung eines Kalibrierungswerts kalibriert wird, der aus einer Mehrzahl von Referenzblutzuckerwerten berechnet wird, die von der separaten Blutzuckermessvorrichtung gemessen werden.

2.  Verfahren zur Kalibrierung des Blutzuckerwerts gemäß Anspruch 1, weiterhin umfassend:

Berechnen (S211) eines Bereichs eines eingebbaren Referenzblutzuckerwerts, der im ersten Kalibrierungsmodus eingebbar ist, basierend auf dem von der Vorrichtung zur kontinuierlichen Blutzuckermessung in dem Kalibrierungszeitraum gemessenen Blutzuckerwert und dem ersten Schwellenwertbereich; und
Steuern (S213) der Aktivierung einer Scroll-Funktion zur Eingabe des Referenzblutzuckerwerts innerhalb des berechneten Bereichs des eingebbaren Referenzblutzuckerwerts auf einer Benutzeroberfläche,
wobei der Referenzblutzuckerwert über die aktivierte Scroll-Funktion eingegeben wird.

3.  Verfahren zur Kalibrierung des Blutzuckerwerts gemäß Anspruch 2, wobei ein Benutzerbefehl zum Auswählen des zweiten Kalibrierungsmodus über die Benutzeroberfläche eingegeben wird, wenn der Referenzblutzuckerwert außerhalb des Bereichs des eingebbaren Blutzuckerwerts der aktivierten Scroll-Funktion liegt.

4.  Verfahren zur Kalibrierung des Blutzuckerwerts gemäß Anspruch 3, wobei die Mehrzahl der zu unterschiedlichen Zeitpunkten gemessenen Referenzblutzuckerwerte im zweiten Kalibrierungsmodus über die Benutzeroberfläche eingegeben wird.

5.  Verfahren zur Kalibrierung des Blutzuckerwerts gemäß einem der Ansprüche 1 bis 4, wobei das Auswählen (S150) des ersten Kalibrierungsmodus oder des zweiten Kalibrierungsmodus umfasst:

Aktivieren (S231) eines Eingabefensters zur Eingabe des im Kalibrierungszeitraum gemessenen Referenzblutzuckers;
Bestimmen (S233), ob die Differenz zwischen dem Blutzuckerwert der Vorrichtung zur kontinuierlichen Blutzuckermessung und dem von der separaten Blutzuckermessvorrichtung gemessen Referenzblutzuckerwert außerhalb des ersten Schwellenwertbereichs liegt; und
wenn die Differenz zwischen dem Blutzuckerwert der Vorrichtung zur kontinuierlichen Blutzuckermessung und dem von der separaten Blutzuckermessvorrichtung gemessenen Referenzblutzuckerwert innerhalb des ersten Schwellenwertbereichs liegt, Auswählen (S235) des ersten Kalibrierungsmodus als Kalibrierungsmodus, und
wenn die Differenz zwischen dem Blutzuckerwert der Vorrichtung zur kontinuierlichen Blutzuckermessung und dem von der separaten Blutzuckermessvorrichtung gemessenen Referenzblutzuckerwert außerhalb des ersten Schwellenwertbereichs liegt, Auswählen (S237) des zweiten Kalibrierungsmodus als Kalibrierungsmodus.

6.  Verfahren zur Kalibrierung des Blutzuckerwerts gemäß einem der Ansprüche 2 bis 5, weiterhin umfassend, wenn die Differenz zwischen dem Blutzuckerwert der Vorrichtung zur kontinuierlichen Blutzuckermessung und dem von der separaten Blutzuckermessvorrichtung gemessenen Referenzblutzuckerwert außerhalb eines zweiten Schwellenwertbereichs liegt, Ausgeben (S330) eines Alarms an einen Benutzer.

7.  Verfahren zur Kalibrierung des Blutzuckerwerts gemäß einem der Ansprüche 2 bis 6, weiterhin umfassend:

Überwachen der Zeitpunkte, zu denen die Mehrzahl von Referenzblutzuckerwerten im zweiten Kalibrierungsmodus eingegeben wird, und
Ausgeben eines Alarms an einen Benutzer, wenn eine Differenz zwischen den Zeitpunkten, zu denen die Mehrzahl von Referenzblutzuckerwerten eingegeben wird, eine vorbestimmte Schwellenwertzeitdifferenz überschreitet.

**Revendications**

1. Procédé d'étalonnage de valeur de glycémie mesurée par un dispositif de mesure continue du glucose sanguin, ledit procédé comprenant :

   l'extraction (S130) d'informations d'une valeur de glycémie mesurée par un dispositif de mesure continue du glucose sanguin pendant une période d'étalonnage ;
   la mesure d'une valeur de glycémie de référence au moyen d'un dispositif séparé de mesure du glucose sanguin, lequel est un dispositif séparé du dispositif de mesure continue du glucose sanguin pendant la période d'étalonnage ;
   le calcul d'une différence entre la valeur de glycémie de référence et la valeur de glycémie du dispositif de mesure du glucose sanguin ;
   la sélection (S150) d'un premier mode d'étalonnage ou d'un deuxième mode d'étalonnage en tant que mode d'étalonnage afin d'étalonner la valeur de glycémie du dispositif de mesure continue du glucose sanguin selon que la différence entre la valeur de glycémie de référence et la valeur de glycémie du dispositif de mesure du glucose sanguin est extérieure à, ou comprise dans une première plage seuil ; et
   l'étalonnage (S170) de la valeur de glycémie du dispositif de mesure continue du glucose sanguin au moyen de la valeur de glycémie de référence en fonction du mode d'étalonnage sélectionné,
   où, si la différence entre la valeur de glycémie du dispositif de mesure continue du glucose sanguin et la valeur de glycémie de référence mesurée par le dispositif séparé de mesure du glucose sanguin est comprise dans la première plage seuil, le premier mode d'étalonnage est sélectionné et la valeur de glycémie du dispositif de mesure continue du glucose sanguin est étalonnée au moyen de la valeur de glycémie de référence dans le premier mode d'étalonnage,
   où, si la différence entre la valeur de glycémie du dispositif de mesure continue du glucose sanguin et la valeur de glycémie de référence mesurée par le dispositif séparé de mesure du glucose sanguin est extérieure à la première plage seuil, le deuxième mode d'étalonnage est sélectionné et la valeur de glycémie du dispositif de mesure continue du glucose sanguin est étalonnée au moyen d'une valeur d'étalonnage calculée à partir d'une pluralité de valeurs de glycémie de référence mesurées par le dispositif séparé de mesure du glucose sanguin dans le deuxième mode d'étalonnage.

2. Procédé d'étalonnage de valeur de glycémie selon la revendication 1, comprenant en outre :

   le calcul (S211) d'une plage d'une valeur de glycémie de référence saisissable, laquelle peut être entrée dans le premier mode d'étalonnage, sur la base de la valeur de glycémie mesurée par le dispositif de mesure du glucose sanguin pendant la période d'étalonnage et de la première plage seuil; et
   la commande (S213) d'activation d'un défilement pour entrer la valeur de glycémie de référence dans la plage calculée de valeur de glycémie de référence saisissable sur un écran d'interface d'utilisateur,
   où la valeur de glycémie de référence est entrée par le défilement activé.

3. Procédé d'étalonnage de valeur de glycémie selon la revendication 2, où une instruction d'utilisateur pour sélectionner le deuxième mode d'étalonnage est entrée par l'écran d'interface d'utilisateur si la valeur de glycémie de référence est extérieure à la plage de valeur de glycémie de référence saisissable du défilement activé.

4. Procédé d'étalonnage de valeur de glycémie selon la revendication 3, où la pluralité de valeurs de glycémie de référence mesurées à différents moments sont entrées par l'écran d'interface d'utilisateur dans le deuxième mode d'étalonnage.

5. Procédé d'étalonnage de valeur de glycémie selon l'une des revendications 1 à 4, où la sélection (S150) du premier mode d'étalonnage ou du deuxième mode d'étalonnage comprend :

   l'activation (S231) d'une fenêtre d'entrée pour saisir la glycémie de référence mesurée pendant la période d'étalonnage ;
   la détermination (S233) si la différence entre la valeur de glycémie du dispositif de mesure continue du glucose sanguin et la valeur de glycémie de référence mesurée par le dispositif séparé de mesure du glucose sanguin est extérieure à la première plage seuil ; et,
si la différence entre la valeur de glycémie du dispositif de mesure continue du glucose sanguin et la valeur de glycémie de référence mesurée par le dispositif séparé de mesure du glucose sanguin est comprise dans la première plage seuil, la sélection (S235) du premier mode d'étalonnage en tant que mode d'étalonnage, et, si la différence entre la valeur de glycémie du dispositif de mesure continue du glucose sanguin et la valeur de glycémie de référence mesurée par le dispositif séparé de mesure du glucose sanguin est extérieure à la première plage seuil, la sélection (S237) du deuxième mode d'étalonnage en tant que mode d'étalonnage.

6. Procédé d'étalonnage de valeur de glycémie selon l'une des revendications 2 à 5, comprenant en outre, si la différence entre la valeur de glycémie du dispositif de mesure continue du glucose sanguin et la valeur de glycémie de référence mesurée par le dispositif séparé de mesure du glucose sanguin est extérieure à une deuxième plage seuil, la transmission (S330) d'une alerte à un utilisateur.

7. Procédé d'étalonnage de valeur de glycémie selon l'une des revendications 2 à 6, comprenant en outre :

des temps de surveillance où la pluralité de valeurs de glycémie de référence sont entrées dans le deuxième mode d'étalonnage, et,
si différence entre les temps où la pluralité de valeurs de glycémie de référence sont entrées dépasse une différence seuil prédéterminée, la transmission d'une alerte à l'utilisateur.

Fig. 1

10

30

Fig. 2

Fig. 3

| 110 | 130 | 150 |
| USER INTERFACE | CALIBRATION MANAGEMENT UNIT | STORING UNIT |

| 170 | 190 |
| CALIBRATION MODE SELECTING UNIT | CALIBRATION UNIT |

Fig. 4

Fig. 5

Fig. 6

PLEASE INPUT REFERENCE
BLOOD GLUCOSE VALUE

INPUT OTHER THAN
THRESHOLD RANGE

CONFIRM

(a)

PLEASE INPUT REFERENCE
BLOOD GLUCOSE VALUE

104

102

100

98

96

94

S

$+\dfrac{TH_1}{2}$

Vt

$-\dfrac{TH_1}{2}$

(b)

Fig. 7

START

S231

INPUTTING REFERENCE
BLOOD GLUCOSE VALUE

S233

NO $|V_S - Yt| < TH_1?$

S237

SELECTING SECOND
CALIBRATION MODE

YES

S235

SELECTING FIRST
CALIBRATION MODE

INPUT WINDOW
ACTIVATION

S239

END

Fig. 8

PLEASE INPUT REFERENCE
BLOOD GLUCOSE VALUE

[            ]

CONFIRM

(a)

PLEASE INPUT REFERENCE
BLOOD GLUCOSE VALUE

[ **151** ]

PLEASE INPUT ADDITIONAL
REFERENCE BLOOD
GLUCOSE LEVEL

[ **156** ]

[ **154** ]

CONFIRM

(b)

Fig. 9

```
                    ┌──────────────┐
                    │    START     │
                    └──────┬───────┘
                           │
                           ▼
        NO           ╱─────────────╲
   ┌─────────────── ◁  |V_S −Yt| > TH_2 ?  ▷ ──── S310
   │                 ╲─────────────╱
   │                       │ YES
   │                       ▼
   │           ┌───────────────────────────┐
   │           │ PROVIDING ATTACHMENT ALARM │ ──── S330
   │           └───────────┬───────────────┘
   │                       │
   │                       ▼
   │   NO            ╱─────────────╲
   │  ┌──────────── ◁  |t_S −Y_2| > TH_T ?  ▷ ──── S350
   │  │              ╲─────────────╱
   │  │                    │ YES
   │  │                    ▼
   │  │        ┌───────────────────────────┐
   │  │        │   PROVIDING CONTINUOUS    │ ──── S370
   │  │        │       INPUT ALARM         │
   │  │        └───────────┬───────────────┘
   │  │                    │
   │  └────────────────────┤
   └───────────────────────┤
                           ▼
                    ┌──────────────┐
                    │     END      │
                    └──────────────┘
```

Decision S310: $|V_S - Yt| > TH_2$ ?

Decision S350: $|t_S - Y_2| > TH_T$ ?

Fig. 10

PLEASE CHECK WHETHER
BODY ATTACHABLE UNIT
IS DETACHED FROM BODY

CONFIRM

(a)

PLEASE CONTINUOUSLY
MEASURE REFERENCE
BLOOD GLUCOSE VALUE
USING BLOOD GLUCOSE
MEASUREMENT DEVICE
WITHIN 10 MINUTES

CONFIRM

(b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160081597 A1